(19) Europäisches Patentamt
European Patent Office
Office européen des brevets

(11) Veröffentlichungsnummer : **0 340 581 B1**

# (12) EUROPÄISCHE PATENTSCHRIFT

(45) Veröffentlichungstag der Patentschrift :
**18.03.92 Patentblatt 92/12**

(51) Int. Cl.$^5$ : **C07C 17/12,** C07C 25/02

(21) Anmeldenummer : **89107344.7**

(22) Anmeldetag : **24.04.89**

## (54) Verfahren zur Kernchlorierung von aromatischen Kohlenwasserstoffen.

(30) Priorität : **06.05.88 DE 3815537**
**15.07.88 DE 3824068**

(43) Veröffentlichungstag der Anmeldung :
**08.11.89 Patentblatt 89/45**

(45) Bekanntmachung des Hinweises auf die Patenterteilung :
**18.03.92 Patentblatt 92/12**

(84) Benannte Vertragsstaaten :
**BE CH DE FR GB IT LI**

(56) Entgegenhaltungen :
**EP-A- 0 126 669**
**EP-A- 0 292 824**

(73) Patentinhaber : **BAYER AG**
**W-5090 Leverkusen 1 Bayerwerk (DE)**

(72) Erfinder : **Mais, Franz-Josef Dr.**
**Gustav-Poensgen-Strasse 23**
**W-4000 Düsseldorf (DE)**
Erfinder : **Fiege, Helmut Dr.**
**Walter-Flex-Strasse 23**
**W-5090 Leverkusen (DE)**

**EP 0 340 581 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Kernchlorierung von aromatischen Kohlenwasserstoffen in Gegenwart von Friedel-Crafts-Katalysatoren und in Gegenwart von Co-Katalysatoren in flüssiger Phase.

Die Umsetzung von aromatischen Kohlenwasserstoffen, wie Toluol, in flüssiger Phase mit gasförmigem Chlor zu kernsubstituierten Chlorderivaten, wie Monochlortoluol, ist bekannt (Ullmanns Enzyklopädie der technischen Chemie, 4. Auflage, Band 9, Seite 499f.). Man führt diese Chlorierung im allgemeinen in Gegenwart von Friedel-Crafts-Katalysatoren, wie Eisen(III)-chlorid, Antimonchloriden oder Aluminiumchlorid, durch. Das erhaltene Chlorierungsprodukt ist gewöhnlich eine Mischung aus isomeren monochlorierten und polychlorierten Verbindungen. Bei Verwendung von $FeCl_3$ erhält man beispielsweise aus Toluol ein Gemisch aus Monochlortoluolen und Dichlortoluolen; in der Monochlortoluolfraktion ist das Hauptprodukt o-Chlortoluol neben p-Chlortoluol und einem geringen Anteil an m-Chlortoluol.

Da besonders p-Chloralkylbenzole, wie p-Chlortoluol, wertvolle Zwischenprodukte darstellen, hat es in der Vergangenheit nicht an Versuchen gefehlt, die Chlorierung so zu lenken, daß das Verhältnis von o- zu p-Chloralkylbenzolen erniedrigt wird, d.h., man versucht, Bedingungen zu finden, die die Bildung von p-Chloralkylbenzolen begünstigen.

Aus US 3.226.447 ist bekannt, daß durch Zusatz von Schwefelverbindungen mit zweiwertigem Schwefel zum Friedel-Crafts-Katalysator bei der Chlorierung von Toluol ein o/p-Verhältnis von 1,2 erhalten werden kann. Nachteilig bei diesem Verfahren ist die Tatsache, daß man dieses wenig günstige Verhältnis nur bei Anwendung von Antimonsalzen als Friedel-Crafts-Katalysatoren erreicht. Nachteilig ist weiterhin, daß die erforderlichen Mengen der Katalysatorkomponenten gemäß dem dortigen Beispiel 16 sehr hoch liegen, nämlich bei 1 Gew.-% für jeden der beiden katalytischen Zusätze. Wie das o/p-Verhältnis mit einem Wert von >1 zeigt, entsteht hierbei immer noch mehr o- als p-Chlortoluol.

In DE-OS 15 43 020 und US. 4.031.144 wird ebenfalls die Chlorierung von Toluol beispielsweise mit $FeCl_3$ und $S_2Cl_2$ beschrieben. Das erhaltene Verhältnis von o/p = 1,03-1,10 ist immer noch unbefriedigend hoch.

In US 4.031.147, US 4.069.263, US 4.069.264 and US 4.250.122 ist die Chlorierung von Toluol mit Friedel-Crafts-Katalysatoren unter Zusatz von Thianthrenen oder substituierten Thianthrenen beschrieben. Die günstigsten erreichbaren o/p-Verhältnisse liegen bei etwa 0,7, werden jedoch entweder nur durch die Verwendung von Antimonsalzen oder im Falle der Verwendung von Eisensalzen nur bei sehr niedrigen Reaktionstemperaturen von etwa 0° erhalten. Beides ist technisch ausgesprochen ungünstig. So wird die co-katalytische Wirkung der Thianthrene beim Einsatz von Antimonsalzen durch Eisenspuren stark behindert, was in der Technik nur schwer zu vermeiden ist. Zudem ist die Reaktion so stark exotherm, daß eine Abführung der Wärme bei etwa 0° durch Solekühlung sehr aufwendig wird. Ferner werden die Thianthrene unter üblichen Reaktionsbedingungen bereits von allgegenwärtigen Wasserspuren zerstört und verlieren somit ihre Wirksamkeit.

Weiterhin ist aus US 4.289.916, EP 63 384 und EP 173 222 die Chlorierung von Toluol in Gegenwart von Lewis-Säuren und Phenoxathiinen bekannt. Das nach Beispiel 1 von EP 173 222 erreichbare o/p-Verhältnis von 0,6 wird wiederum nur durch die technisch äußerst ungünstige Verwendung von Antimonchlorid und die hohe Menge von 0,29 Gew.-% an Co-Katalysator erreicht. Bei Verwendung von $FeCl_3$ anstelle von Antimonchlorid erhält man ein o/p-Verhältnis von 0,68, allerdings wiederum nur bei der technisch äußerst ungünstigen niedrigen Reaktionstemperatur von 5°C. Bei einer technisch vorteilhaften Reaktionstemperatur von 50°C steigt das o/p-Verhältnis in Gegenwart von $FeCl_3$ und dem in EP 173 222 beanspruchten Phenoxathiin-Derivat auf 0,88, wie von uns durchgeführte Versuche zeigen (vgl. Beispiel 21). In den genannten US 4.289.916 und EP 63 384 wird ein günstigstes o/p-Verhältnis von etwa 0,8 beschrieben. Auch hier kann das o/p-Verhältnis auf 0,65 gesenkt werden, wenn man anstelle von $FeCl_3$ Antimonchloride und eine Reaktionstemperatur von 20°C, also technisch ungünstige Bedingungen, anwendet. Auch Phenoxathiine werden in Gegenwart von Wasserspuren zerstört.

Aus EP 126 669 ist die Toluol-Chlorierung in Gegenwart von Friedel-Crafts-Katalysatoren und N-substituierten Phenothiazinen bekannt. Das o/p-Verhältnis ist mit 0,84 auch hierbei ungünstig hoch.

Aus EP 112 722, EP 154 236 und EP 248 931 ist die Chlorierung von Toluol in Gegenwart von bestimmten Zeolithen bekannt, wobei unter Zusatz von beispielsweise Halogen-carbonsäurehalogeniden als Moderatoren ein o/p-Verhältnis von etwa 0,3 erreicht wird. Nachteilig an diesem Verfahren sind die erheblichen Mengen von 5 Gew.-% Zeolith und 1 Gew.-% an Moderatoren. Wie eigene Versuche zeigten, muß dieses Ergebnis mit dem erheblichen Nachteil erkauft werden, daß in den erhaltenen Gemischen sehr große Mengen (bis zu 8 Gew.-%) an Benzylchloriden auftreten. Die Bildung von Benzylchloriden stört die nachfolgende übliche destillative Aufarbeitung in ganz außerordentlichem Maße.

Es wurde nun ein Verfahren zur Kernchlorierung von aromatischen Kohlenwasserstoffen der Formel

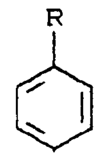 (I),

worin
R geradkettiges oder verzweigtes $C_1$-$C_{12}$-Alkyl oder $C_3$-$C_8$-Cycloalkyl bedeutet,
in Gegenwart von Friedel-Crafts-Katalysatoren und in Gegenwart von Co-Katalysatoren in flüssiger Phase gefunden, das dadurch gekennzeichnet ist, daß man als Co-Katalysatoren 1,6-Benzo-thiazocine einsetzt.

Das Gerüst der 1,6-Benzo-thiazocine wird durch die folgende Formel mit Numerierung dargestellt:

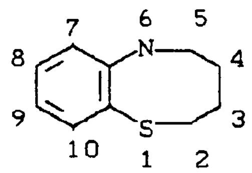 (II).

Die 1,6-Benzo-thiazocine für das erfindungsgemäße Verfahren können durch die folgende Formel gekennzeichnet werden

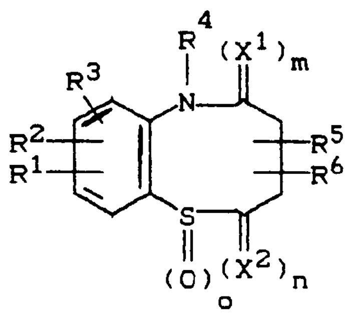 (III),

in der
$R^1$ und $R^2$ unabhängig voneinander Wasserstoff, Hydroxy, Amino, Cyano, Halogen, Nitro, Alkylsulfonyl, Phenylsulfonyl, Alkylsulfoxyl, Phenylsulfoxyl, Tosyl, Mercapto, Carboxyl, Halogenocarbonyl, Carboxyamid, Alkoxycarbonyl, Thiocarboxyamid, Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino bedeuten,
$R^3$ für Wasserstoff oder Chlor steht und weiterhin mit einem der Reste $R^1$ oder $R^2$ und gemeinsam mit den substituierten C-Atomen einen annellierten gesättigten, ungesättigten oder aromatischen isocyclischen oder heterocyclischen 5-8-Ring bilden kann,
$R^4$ Wasserstoff, Halogencarbonyl, Alkyl, Aryl, Heteroaryl, Acyl, Thioacyl oder Alkoxycarbonyl bedeutet,
$X^1$ und $X^2$ unabhängig voneinander für doppelt gebundenen Sauerstoff, Schwefel oder $R^7$-substituierten Stickstoff stehen, wobei $R^7$ den Bedeutungsumfang von $R^4$ mit Ausnahme von Wasserstoff hat,
m, n und o unabhängig voneinander den Wert 0 oder 1 annehmen können und
$R^5$ und $R^6$ unabhängig voneinander an einem oder an zwei der zwischen dem S- und dem N-Atom im 8-Ring befindlichen C-Atome stehen können, sofern diese C-Atome nicht durch $X^1$ bzw. $X^2$ besetzt sind, und den Bedeutungsumfang von $R^1$ bzw. $R^2$ haben, wobei bei benachbarter Substitution auch mit den substituierten C-Atomen ein gesättigter, ungesättigter oder aromatischer isocyclischer oder heterocyclischer 5-8-Ring gebildet werden kann und wobei weiterhin der Bedeutungsumfang des doppelt gebundenen Sauerstoffs oder Schwefels angenommen werden kann.

Als Halogen sei Fluor, Chlor, Brom oder Iod, bevorzugt Fluor, Chlor oder Brom, besonders bevorzugt Fluor oder Chlor, genannt.

Als Alkylreste in den genannten Substituenten seien offenkettige mit 1-16 C-Atomen, bevorzugt mit 1-4 C-Atomen, und cyclische mit 5-8 C-Atomen, bevorzugt mit 5 oder 6 C-Atomen, genannt. Diese Alkylreste können ihrerseits mit $C_1$-$C_4$-Alkyl, bevorzugt mit Methyl oder Ethyl, substituiert sein, so daß man auch in die Reihe der verzweigten Alkylreste gelangt. Diese Alkylreste können weiterhin durch Fluor, Chlor oder Brom ein- oder

mehrfach substituiert sein. Diese Alkylreste können weiterhin durch $C_1$-$C_4$-Alkoxy, bevorzugt mit Methoxy oder Ethoxy, substituiert sein, so daß man in die Reihe der Ether gelangt. Diese Alkylreste können weiterhin durch Phenyl, Naphthyl oder Biphenyl substituiert sein, so daß man in die Reihe der Aralkylreste gelangt. Beispiele für solche Alkylreste sind: Methyl, Ethyl, Propyl, Isopropyl, Butyl, Isobutyl, tert.-Butyl, Amyl, Hexyl, Octyl, Decyl, Dodecyl, Hexadecyl, Cyclopentyl, Methylcyclopentyl, Cyclohexyl, Methylcyclohexyl, Cycloheptyl, Cyclooctyl, Methoxymethyl, Ethoxymethyl, Benzyl, Phenylethyl, Chlormethyl, Dichlormethyl, Trichlormethyl, Trifluormethyl; besonders wichtige Reste sind beispielsweise Methyl, Ethyl, n-Propyl, Benzyl, Trifluormethyl.

Der genannte Bedeutungsumfang für Alkylreste gilt grundsätzlich auch für Alkoxy und Alkylthio; bevorzugt sind Reste mit 1-6 C-Atomen, besonders bevorzugt solche mit 1-4 C-Atomen, wie Methoxy, Ethoxy, tert.-Butoxy, Cyclohexyloxy, Trifluormethoxy, Methylthio, Ethylthio, Cyclohexylthio, Trifluromethylthio, Trichlormethylthio.

Als Arylreste in den obigen Substituenten seien beispielsweise Phenyl, Naphthyl oder Biphenyl genannt, die ihrerseits durch Fluor, Chlor, Brom, Nitro, $C_1$-$C_4$-Alkyl oder $C_1$-$C_4$-Alkoxy substituiert sein können, beispielsweise Phenyl, Naphthyl, Tolyl, Anisyl, Chlorphenyl, Nitrophenyl; besonders wichtig sind beispielsweise Phenyl und Chlorphenyl.

Als Heteroarylreste in den obigen Substituenten seien solche mit 5-9 C-Atomen, bevorzugt mit 5 oder 6 C-Atomen, genannt, die 1-3, bevorzugt 1 oder 2, besonders bevorzugt 1 Heteroatom, im Ring aufweisen. Sie können aromatisch oder nicht aromatisch, bevorzugt jedoch aromatisch, sein. Diese Heteroarylreste können ihrerseits durch Methyl, Ethyl, Fluor oder Chlor substituiert sein. Als Beispiele seien genannt: Pyridyl, Methylpyridyl, Furyl, Pyrrolyl, Imidazolyl.

Bezüglich der Aryloxy-, Heteroaryloxy-, Arylthio- und Heteroarylthioreste gilt analog das oben zu den Alkoxy- und Alkylthioresten Gesagte.

Acylreste innerhalb der obigen Substituenten haben 2-8 C-Atome und sind aliphatisch, bevorzugt mit 2-4 C-Atomen, oder bei der erforderlichen Zahl von C-Atomen aromatisch. Sie können ihrerseits durch oben für Alkylreste bzw. Arylreste genannte Zweitsubstituenten substituiert sein. Als Beispiele seien genannt: Acetyl, Chloracetyl, Trichloracetyl, Trifluoracetyl, Benzoyl, Chlorbenzoyl, Chlorcarbonyl, Formyl.

Für den Fall, daß $R^3$ mit einem der Reste $R^1$ oder $R^2$ und gemeinsam mit den substituierten C-Atomen einen Ring bildet, kann dieser isocyclisch und gesättigt, ungesättigt oder aromatisch sein oder auch durch einen Gehalt an N-, O- und/oder S-Atomen heterocyclisch sein. Solche Ringe haben 5-8, bevorzugt 5 oder 6 Ringglieder und sind an den in Formel (III) gezeigten Benzolkern annelliert. Als Beispiele seien genannt: Benzo, Naphthalino, Thieno, Furano, Pyrrolo, Pyridino, Cyclohexano, Cyclopentano, Oxolano, Dioxolano, bevorzugt Benzo und Cyclohexano.

Als Illustration für erfindungsgemäß einsetzbare 1,6-Benzo-thiazocine dient die folgende, keineswegs erschöpfende Aufzählung:

3,4-Dihydro-2H-1,6-benzothiazocin-5(6H)-on,
3,4-Dihydro-2H-1,6-benzothiazocin-5(6H)-thion,
4-Acetyl-3,4-dihydro-2H-1,6-benzothiazocin-5(6H)-on,
4-Methyl-3,4-dihydro-2H-1,6-benzothiazocin-5(6H)-on,
3-Methyl-3,4-dihydro-2H-1,6-benzothiazocin-5(6H)-on,
2-Methyl-3,4-dihydro-2H-1,6-benzothiazocin-5(6H)-on,
2-Ethyl-3,4-dihydro-2H-1,6-benzothiazocin-5(6H)-on,
2-Propyl-3,4-dihydro-2H-1,6-benzothiazocin-5(6H)-on,
2-Phenyl-3,4-dihydro-2H-1,6-benzothiazocin-5(6H)-on,
4-Methyl-4-acetyl-3,4-dihydro-2H-1,6-benzothiazocin-5(6H)-on,
2,4-Dimethyl-3,4-dihydro-2H-1,6-benzothiazocin-5(6H)-on,
3,4-Tetramethylen-3,4-dihydro-2H-1,6-benzothiazocin-5(6H)-on,
2,3-Tetramethylen-3,4-dihydro-2H-1,6-benzothiazocin-5(6H)-on,
2,3-Tetramethylen-5,6-dihydro-2H-1,6-benzothiazocin-4(3H)-on,
2,3-Tetramethylen-3,4,5,6-tetrahydro-2H-1,6-benzothiazocin,
3,4,5,6-Tetrahydro-2H-1,6-benzothiazocin,
6-Acetyl-3,4,5,6-tetrahydro-2H-1,6-benzothiazocin,
6-Trifluoracetyl-3,4,5,6-tetrahydro-2H-1,6-benzothiazocin,
6-Chloracetyl-3,4,5,6-tetrahydro-2H-1,6-benzothiazocin,
6-Methyl-3,4,5,6-tetrahydro-2H-1,6-benzothiazocin,
6-Ethyl-3,4,5,6-tetrahydro-2H-1,6-benzothiazocin,
6-Acetyl-3,4-dihydro-2H-1,6-benzothiazocin-5(6H)-on,
6-Trifluoracetyl-3,4-dihydro-2H-1,6-benzothiazocin-5(6H)-on,
6-Chloracetyl-3,4-dihydro-2H-1,6-benzothiazocin-5(6H)-on,

6-Methyl-3,4-dihydro-2H-1,6-benzothiazocin-5(6H)-on,
6-Benzyl-3,4-dihydro-2H-1,6-benzothiazocin-5(6H)-on,
2H-Benzothiazocin-3(4H)-5(6H)-dion,
2H-3,4-Dihydro-1,6-benzothiazocin-2,5(6H)-dion,
8-Chlor-3,4-dihydro-2H-1,6-benzothiazocin-5(6H)-on,
8-Methyl-3,4-dihydro-2H-1,6-benzothiazocin-5(6H)-on,
8-Methoxy-3,4-dihydro-2H-1,6-benzothiazocin-5(6H)-on,
9-Methoxy-3,4-dihydro-2H-1,6-benzothiazocin-5(6H)-on,
8-Methoxy-2,3-tetramethylen-3,4-dihydro-2H-1,6-benzothiazocin-5(6H)-on,
8-Fluor-3,4-dihydro-2H-1,6-benzothiazocin-5(6H)-on,
2,3-Dimethyl-3,4-dihydro-2H-1,6-benzothiazocin-5(6H)-on,
2,3-Dimethyl-6-acetyl-3,4-dihydro-2H-1,6-benzothiazocin-5(6H)-on,
8,9-Dimethyl-3,4-dihydro-2H-1,6-benzothiazocin-5(6H)-on,
8,10-Dimethyl-3,4-dihydro-2H-1,6-benzothiazocin-5(6H)-on,
8,10-Dimethyl-3,4,5,6-tetrahydro-2H-1,6-benzothiazocin,
8,10-Dimethyl-2,3-tetramethylen-3,4-dihydro-2H-1,6-benzothiazocin-5(6H)-on,
8,10-Dimethyl-3,4-dihydro-2H-1,6-benzothiazocin-5(6H)-thion,
8,10-Dimethyl-2,3-tetramethylen-3,4-dihydro-2H-1,6-benzothiazocin-5(6H)-thion,
8-Methyl-3,4-dihydro-2H-1,6-benzothiazocin-5(6H)-thion,
8-Trifluormethyl-3,4-dihydro-2H-1,6-benzothiazocin-5(6H)-on,
2-Chlor-3,4-dihydro-2H-1,6-benzothiazocin-5(6H)-on,
2,2-Dimethyl-3,4-dihydro-2H-1,6-benzothiazocin-5(6H)-on,
1-Oxo-3,4-dihydro-2H-1,6-benzothiazocin-5(6H)-on,
1-Oxo-3,4,5,6-tetrahydro-2H-1,6-benzothiazodin,
2H-1,6-dibenzo[b,f]thiazocin-5(6H)-on,
5,6-Dihydro-2H-1,6-dibenzo[b,f]thiazocin und 3,4-Dihydro-2H-1,6-2,3-napthothiazocin-5(6H)-on,
bevorzugt:
3,4-Dihydro-2H-1,6-benzothiazocin-5(6H)-on,
2-Methyl-3,4-dihydro-2H-1,6-benzothiazocin-5(6H)-on,
2,4-Dimethyl-3,4-dihydro-2H-1,6-benzothiazocin-5(6H)-on,
3,4-Tetramethylen-3,4-dihydro-2H-1,6-benzothiazocin-5(6H)-on,
2,3-Tetramethylen-3,4-dihydro-2H-1,6-benzothiazocin-5(6H)-on,
2,3-Tetramethylen-3,4,5,6-tetrahydro-2H-1,6-benzothiazocin,
3,4,5,6-Tetrahydro-2H-1,6-benzothiazocin,
6-Trifluoracetyl-3,4,5,6-tetrahydro-2H-1,6-benzothiazocin,
6-Acetyl-3,4-dihydro-2H-1,6-benzothiazocin-5(6H)-on,
6-Methyl-3,4-dihydro-2H-1,6-benzothiazocin-5(6H)-on,
8-Methyl-3,4-dihydro-2H-1,6-benzothiazocin-5(6H)-on,
6-Acetyl-3,4,5,6-tetrahydro-benzothiazocin,
2-Phenyl-3,4-dihydro-2H-1,6-benzothiazocin-5(6H)-on,
4-Methyl-3,4-dihydro-2H-1,6-benzothiazocin-5(6H)-on,
8-Methoxy-3,4-dihydro-2H-1,6-benzothiazocin-5(6H)-on,
8-Methoxy-2,3-tetramethylen-3,4-dihydro-2H-1,6-benzothiazocin-5(6H)-on,
2,3-Dimethyl-3,4-dihydro-2H-1,6-benzothiazocin-5(6H)-on,
8,10-Dimethyl-3,4-dihydro-2H-1,6-benzothiazocin-5(6H)-on,
8,10-Dimethyl-3,4,5,6-tetrahydro-2H-1,6-benzothiazocin,
8,10-Dimethyl-2,3-tetramethylen-3,4-dihydro-2H-1,6-benzothiazocin-5(6H)-on und
3,4-Dihydro-2H-1,6-benzothiazocin-5(6H)-thion.

Bevorzugte 1,6-Benzo-thiazocine sind solche der Formeln

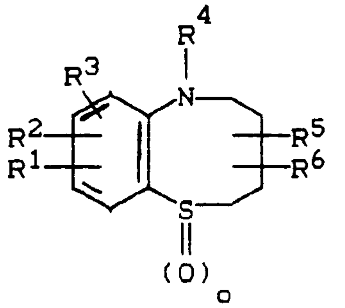

(IV)

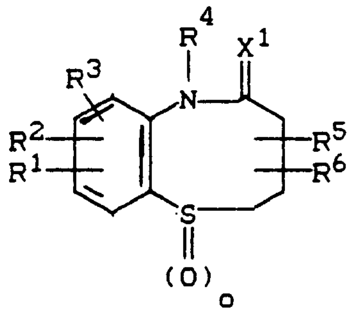

(V)

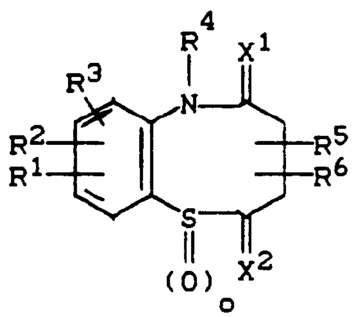

(VI),

in denen
$R^1$ bis $R^6$, $X^1$, $X^2$ und o den obengenannten Bedeutungsumfang haben.

In besonders bevorzugter Weise werden 1,6-Benzo-thiazocine der Formeln (IV) und (V) eingesetzt.

In ganz besonders bevorzugter Form werden 1,6-Benzothiazocine der Formel (V) eingesetzt.

In weiterhin bevorzugter Form werden 1,6-Benzo-thiazocine der Formel (III) eingesetzt, in denen der Index o den Wert Null annimmt.

In weiterhin bevorzugter Form werden 1,6-Benzo-thiazocine der Formel (III) eingesetzt, in denen anstelle von $R^1$, $R^2$ und $R^3$ die Reste $R^{11}$, $R^{12}$ bzw. $R^{13}$ treten, von denen
$R^{11}$ und $R^{12}$ unabhängig voneinander Wasserstoff, Halogen, Nitro, Alkyl, Aryl, Alkoxy, Aryloxy, Acyloxy, Alkylthio, Arylthio, Acyl oder Thioacyl bedeuten und
$R^{13}$ für Wasserstoff oder Chlor steht und weiterhin mit einem der Reste $R^{11}$ und $R^{12}$ und gemeinsam mit den substituierten C-Atomen einen annellierten gesättigten isocyclischen 5-7-Ring oder einen annellierten Benzolring bilden kann.

In ganz besonders bevorzugter Weise werden 1,6-Benzothiazocine der Formel (III) eingesetzt, in denen anstelle von $R^{11}$, $R^{12}$ und $R^{13}$ die Reste $R^{21}$, $R^{22}$ bzw. $R^{23}$ treten, von denen
$R^{21}$ und $R^{22}$ unabhängig voneinander Wasserstoff, $C_1$-$C_4$-Alkyl, $C_1$-$C_4$-Alkoxy, Fluor oder Chlor bedeuten und
$R^{23}$ für Wasserstoff oder Chlor steht und weiterhin mit einem der Reste $R^{21}$ oder $R^{22}$ und gemeinsam mit den substituierten C-Atomen einen annellierten Cyclopentan-, Cyclohexan- oder Benzolring bilden kann.

Weitere bevorzugte 1,6-Benzo-thiazocine sind solche der Formel (III), in denen an die Stelle von $R^4$ der Rest $R^{14}$ tritt, der die Bedeutung Wasserstoff, $C_1$-$C_4$-Alkyl, Phenyl, $C_1$-$C_4$-Acyl oder $C_2$-$C_4$-Alkoxycarbonyl hat.

Besonders bevorzugt sind solche 1,6-Benzo-thiazocine der Formel (III), in denen an die Stelle von $R^{14}$ der Rest $R^{24}$ mit der Bedeutung Wasserstoff, $C_1$-$C_2$-Alkyl, Benzyl, Phenyl, Formyl, Acetyl, Trifluoracetyl, Chlorcarbonyl oder Propionyl tritt.

Weitere bevorzugte 1,6-Benzo-thiazocine sind solche der Formel (III), in denen an die Stelle von $R^5$ und $R^6$ die Reste $R^{15}$ bzw. $R^{16}$ treten, die die Bedeutung Wasserstoff, $C_1$-$C_4$-Alkyl, $C_1$-$C_4$-Alkoxy, $C_1$-$C_4$-Acyl, Phenyl, Fluor oder Chlor haben und wobei weiterhin bei benachbarter Substitution beide Reste gemeinsam mit den substituierten C-Atomen einen Cyclopentan-, Cyclohexan- oder Benzolring bilden können.

Die erfindungsgemäß einzusetzenden 1,6-Benzo-thiazocine können nach bekannten Verfahren hergestellt werden, beispielsweise durch Umsetzung von gegebenenfalls substituierten Aminothiophenolen mit γ-Butyrolactonen (DE-AS 15 45 805, DE-AS 15 45 806, US 3.155.649) oder durch Ringerweiterung von Benzothiepinen (GB 1.112.681, US 3.311.615).

Als Beispiele für die erfindungsgemäß im Kern zu chlorierenden aromatischen Kohlenwasserstoffe der Formel (I) seien genannt: Toluol, Ethylbenzol, Propylbenzol, Cumol, tert.-Butylbenzol und Phenylcyclohexan; besonders wichtig ist das Verfahren für die Kernchlorierung von Toluol.

Das erfindungsgemäße Verfahren wird in flüssiger Phase durchgeführt, wobei der aromatische Kohlenwasserstoff in flüssiger (geschmolzener) Form oder gegebenenfalls in Verdünnung mit einem inerten Lösungsmittel eingesetzt werden kann. Geeignete Lösungsmittel sind solche, die durch Chlor unter den Bedingungen einer Kernchlorierung nicht angegriffen werden und dem Fachmann hierfür bekannt sind, wie Methylenchlorid, Chloroform, Tetrachlorkohlenstoff, Essigsäure. In bevorzugter Weise wird ohne Lösungsmittel gearbeitet.

Als Chlorierungsmittel für das erfindungsgemäße Verfahren wird vorzugsweise Chlor verwendet. Das Chlor kann flüssig oder gasförmig in das Reaktionsgemisch eingeleitet werden. Bevorzugt wird gasförmiges Chlor eingesetzt.

Es können jedoch auch andere Chlorierungsmittel verwendet werden, die, wie beispielsweise Sulfurylchlorid, unter den Reaktionsbedingungen Chlor abgeben.

Der Wassergehalt der Reaktionsmischung ist im Prinzip nicht kritisch, solange der verwendete Friedel-Crafts-Katalysator nicht völlig desaktiviert wird. Daher ist es bevorzugt, die Einsatzstoffe nicht besonders zu trocknen, sondern sie in dem Zustand zu verwenden, in dem sie üblicherweise in der Praxis der technischen Chemie vorkommen. Jedoch ist es erfindungsgemäß ebenso möglich, einige oder alle Einsatzstoffe zu trocknen. Im allgemeinen sollte der Wassergehalt nicht über der Sättigungsgrenze der Einsatzstoffe liegen. Bevorzugt beträgt der Wassergehalt der Reaktionsmischung bis zu 250 ppm, besonders bevorzugt bis zu 150 ppm, ganz besonders bevorzugt bis zu 100 ppm.

Die erfindungsgemäß durchzuführende Kernchlorierung kann grundsätzlich bei einer Temperatur vom Erstarrungspunkt bis zum Siedepunkt des Reaktionsgemisches durchgeführt werden. Im allgemeinen liegt die Reaktionstemperatur bei 0-100°C, bevorzugt 20-80°C, besonders bevorzugt 40-60°C.

Der Reaktionsdruck kann normal, vermindert oder erhöht sein und ist grundsätzlich unkritisch. Wegen der kostengünstigen Durchführung ist Normaldruck bevorzugt. Erhöhter Druck kann beispielsweise dann angezeigt sein, wenn oberhalb des Siedepunktes eines tiefsiedenden Lösungsmittels gearbeitet werden soll; in diesem Fall kann beispielsweise unter dem sich automatisch einstellenden Eigendruck des Reaktionsgemisches gearbeitet werden. Der Chlorierungsgrad des Reaktionsgemisches liegt bevorzugt nicht höher als 1, bezogen auf den zu chlorierenden aromatischen Kohlenwasserstoff. Höhere Chlorierungsgrade sind möglich, aber gewöhnlich nicht vorteilhaft, da sie zur Bildung unerwünschter mehrfach chlorierter Produkte führen. Chlor bzw. eine chlorabgebende Substanz werden daher beispielsweise in einer Menge von 0,8-1,1, bevorzugt 0,8-1,0 Mol pro Mol des aromatischen Kohlenwasserstoffs eingesetzt.

Friedel-Crafts-Katalysatoren für das erfindungsgemäße Verfahren sind alle bekannten, beispielsweise Antimonchloride, Antimonoxichlorid, Aluminiumchlorid, Eisen(II)-chlorid, Eisen(III)-chlorid, Tellurchloride, Molybdänchloride, Wolframchloride, Titanchloride, Zinkchlorid, Zinnchloride, Borchlorid und/oder Bortrifluorid. Es können jedoch auch Elemente und Elementverbindungen, die während der Chlorierung einen Friedel-Crafts-Katalysator (Lewis-Säure) bilden, eingesetzt werden. Beispielsweise die elementaren Metalle oder Halbmetalle Antimon, Eisen, Blei, Zinn, Zink, Molybdän, Tellur und Aluminium oder deren Oxide, Sulfide, Carbonyle oder Salze (beispielsweise Carbonate oder ähnliche); genannt seien beispielsweise Antimonoxide, Eisenoxide, Eisensulfide, Bleisulfide, Zinnsulfide, Zinksulfide, Eisencarbonyle, Molybdäncarbonyle und/oder Borphosphat. Anstelle der erwähnten Chloride können auch die Bromide, gegebenenfalls auch die Fluoride oder Iodide der genannten Elemente eingesetzt werden. Bevorzugte Friedel-Crafts-Katalysatoren sind Antimonchloride, Aluminiumchloride, Eisen, Eisenoxide, Eisensulfide, Eisencarbonyle und/oder Eisen(III)-chlorid. Besonders bevorzugt ist Eisen(III)-chlorid.

Die Mengen des Friedel-Crafts-Katalysators oder eines Gemisches mehrerer von ihnen können in weiten Grenzen variiert werden. So ist bereits bei einem Zusatz von 0,0005 Gew.-% eine Katalysatorwirkung erkennbar; andererseits können auch 5 Gew.-% oder mehr des Friedel-Crafts-Katalysators zugesetzt werden, jedoch

bieten solche hohen Mengen im allgemeinen keinen Vorteil, bringen aber gegebenenfalls bei der Aufarbeitung Schwierigkeiten. Üblicherweise wird der Friedel-Crafts-Katalysator in einer Menge von 0,001-0,5 Gew.-%, bevorzugt 0,01-0,1 Gew.-% eingesetzt. Alle Mengenangaben sind auf die Menge des eingesetzten aromatischen Kohlenwasserstoffs bezogen.

Die erfindungsgemäß einsetzbaren Co-Katalysatoren umfassen neben den obengenannten Substanzen alle Substanzen, die unter den Reaktionsbedingungen Verbindungen oder Gemische von Verbindungen bilden können, die unter die obengenannten Formeln (III) bis (VI) fallen. Das sind beispielsweise solche Verbindungen, die in dem 8-Ring ein- oder mehrfach ungesättigt sind. Weiterhin sind es offenkettige Vorstufen, die unter den erfindungsgemäßen Bedingungen den Ringschluß eingehen und damit in erfindungsgemäße Co-Katalysatoren übergehen. Weiterhin einsetzbar sind alle Substanzen, die durch Reaktion der zuvor genannten erfindungsgemäßen Co-Katalysatoren mit Chlor oder Chlorwasserstoff unter den Reaktionsbedingungen der Chlorierung gebildet werden können. Hier seien beispielsweise die Hydrochloride der obengenannten Co-Katalysatoren genannt.

Es ist weiterhin möglich, die Co-Katalysatoren in Kombination mit anderen, nicht als Co-Katalysatoren beanspruchten Elementen oder Verbindungen im erfindungsgemäßen Verfahren einzusetzen. Die Co-Katalysatoren können sowohl einzeln als auch im Gemisch mehrerer von ihnen eingesetzt werden. Die Mengen, in denen die erfindungsgemäßen Co-Katalysatoren eingesetzt werden, können in weiten Grenzen variieren. Mengen unter 0,0001 Gew.-% sind jedoch weniger vorteilhaft, da dann die co-katalytische Wirkung nachläßt. Es können sogar Mengen von 5 Gew.-% oder mehr an Co-Katalysator zugesetzt werden, jedoch bieten diese hohen Mengen im allgemeinen keinen Vorteil, verursachen aber gegebenenfalls Aufarbeitungsprobleme. Die erfindungsgemäßen Co-Katalysatoren werden daher im allgemeinen in einer Menge von 0,0001-0,5 Gew.-%, bevorzugt 0,0005-0,1 Gew.-%, besonders bevorzugt 0,0005-0,0075 Gew.-%, bezogen auf den eingesetzten aromatischen Kohlenwasserstoff, eingesetzt.

Das Molverhältnis des Gemisches aus Friedel-Crafts-Katalysator(en) und Co-Katalysator(en) kann im erfindungsgemäßen Verfahren in weiten Grenzen variiert werden. Im allgemeinen ist es vorteilhaft, den Co-Katalysator nicht in zu großem Überschuß gegenüber dem Friedel-Crafts-Katalysator einzusetzen. Ebenso ist es im allgemeinen vorteilhafter, auch den Überschuß des Friedel-Crafts-Katalysators nicht zu groß anzusetzen. Erfindungsgemäß ist ein molares Verhältnis von Friedel-Crafts-Katalysator zu Co-Katalysator von 100:1-1:10, bevorzugt 75:1-1:4, besonders bevorzugt 50:1-1:2.

Für die praktische Durchführung des erfindungsgemäßen Verfahrens ist die Reihenfolge der Zugabe der einzelnen Komponenten des Reaktionsgemisches beliebig. Hierbei läßt sich das Verfahren sowohl kontinuierlich als auch diskontinuierlich durchführen. Eine beispielhafte Ausführungsform ist die folgende:

Der gewünschte aromatische Kohlenwasserstoff, beispielsweise Toluol, wird vorgelegt und auf die gewünschte Temperatur (beispielsweise 50°C) gebracht. Dann gibt man in beliebiger Reihenfolge die gewünschten Mengen an Friedel-Crafts-Katalysator(en) und Co-Katalysator(en) zu und leitet unter weitgehender Konstanthaltung der Temperatur Chlor gasförmig bis zum gewünschten Chlorierungsgrad ein. Anschließend wird das Gemisch in üblicher Weise durch Destillation aufgearbeitet.

Eine weitere beispielhafte Ausführungsform ist die folgende:

Man stellt eine Mischung aus Alkylbenzol mit den gewünschten Anteilen an Katalysator und Co-Katalysator her und bringt diese auf die gewünschte Reaktionstemperatur. Dann wird Chlorierungsmittel bis zum gewünschten Chlorierungsgrad eingeleitet. Die Aufarbeitung kann auch hier in üblicher Weise durch Destillation erfolgen.

Ein weitere Ausführungsform ist die folgende:

Man stellt eine Lösung von Katalysator und Co-Katalysator in dem Alkylbenzol her und führt diese einer kontinuierlich arbeitenden Chlorierapparatur zu. Man leitet ebenfalls kontinuierlich ein Chlorierungsmittel so schnell ein, daß der gewünschte Chlorierungsgrad erreicht wird. Auch hier kann die kontinuierlich anfallende Reaktionsmischung in üblicher Weise durch Destillation aufgearbeitet werden.

Im Gegensatz zum erfindungsgemäßen Verfahren hatten die bisher bekannten Heterocyclen zur Steuerung der o/p-Selektivität immer eine andere Struktur, nämlich die Form von drei linear anellierten 6-Ringen.

Beim erfindungsgemäßen Verfahren ist es überraschend, daß die erfindungsgemäßen Co-Katalysatoren eine so ausgeprägte Selektionswirkung auf das o/p-Verhältnis haben, daß überwiegend die p-Verbindung gebildet wird. Ausgesprochen überraschend und überaus vorteilhaft ist ferner die Tatsache, daß die erfindungsgemäßen Co-Katalysatoren gerade mit dem technisch außerordentlich günstigen und wünschenswerten Friedel-Crafts-Katalysator $FeCl_3$ so gute Ergebnisse liefern.

Weiterhin überraschend ist, daß diese guten Ergebnisse bei technisch sehr vorteilhaften Temperaturen, beispielsweise im Bereich von 40-60°C, erreicht werden. Noch weiterhin überraschend ist es, daß die erfindungsgemäßen Co-Katalysatoren ihre p-selektive Wirkung bereits bei äußerst geringen Konzentrationen zeigen, so daß die notwendigen Mengen an Co-Katalysatoren besonders gering sind. So liegen sie im besonders

bevorzugten Bereich von 0,0005-0,0075 Gew.-% um Zehnerpotenzen niedriger als bei den bisher bekannten Co-Katalysatoren.

Diese Tatsache ist technisch wie ökonomisch und ökologisch außerordentlich vorteilhaft.

Die erfindungsgemäßen Co-Katalysatoren sind in weiterhin günstiger Weise auf einfache Art durch einen einzigen Reaktionsschritt aus technisch verfügbaren Ausgangsmaterialien herstellbar.

Beispiel 1

Man legte unter Rühren 100 Gew.-Teile Toluol in einem Reaktor vor und gab 0,017 Gew.-Teile $FeCl_3$ und 0,0045 Gew.-Teile des Co-Katalysators der Formel

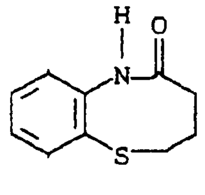

(3,4-Dihydro-2H-1,6-benzothiazocin-5(6H)-on)

zu und erhitzte auf 50°C. Unter weitgehender Konstanthaltung der Temperatur leitete man 93-95 Mol-% Chlor, bezogen auf Toluol, gasförmig im Verlauf von 5 h gleichmäßig ein. Der Restgehalt an Toluol im Reaktionsgemisch betrug 4,3 Gew.-%, das Verhältnis von ortho-Chlortoluol zu para-Chlortoluol (o/p) betrug 0,80.

Beispiel 2

Das Verfahren von Beispiel 1 wurde wiederholt. Man gab jedoch 0,0045 Gew.-Teile des Co-Katalysators der Formel

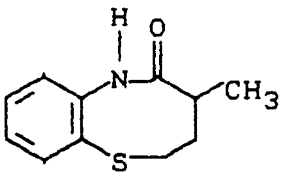

(4-Methyl-3,4-dihydro-2H-1,6-benzothiazocin-5(6H)-on)

zu. Das Resttoluolgehalt betrug 4,6 %, das o/p-Verhältnis 0,94.

Beispiel 3

Das Verfahren von Beispiel 1 wurde wiederholt, man verwendete jedoch statt Toluol 100 Gew.-Teile Ethylbenzol. Der Restgehalt an Ethylbenzol betrug 10,6 Gew.-%, das Verhältnis von ortho-Chlorethylbenzol zu para-Chlorethylbenzol betrug o/p = 0,69.

Beispiel 4

Das Verfahren von Beispiel 1 wurde wiederholt, man verwendete jedoch statt Toluol 100 Gew.-Teile Cumol. Der Restgehalt an Cumol betrug 11,7 Gew.-%, das Verhältnis von ortho-Chlorisopropylbenzol zu para-Chlorisopropylbenzol betrug o/p = 0,41.

Beispiel 5

Das Verfahren von Beispiel 1 wurde wiederholt, man verwendete jedoch statt Toluol 100 Gew.-Teile Cyclohexylbenzol. Der Restgehalt an Cyclohexylbenzol betrug 10,8 Gew.-%, das Verhältnis von ortho-Chlorcyclohexylbenzol zu para-Chlorcyclohexylbenzol betrug o/p = 0,44.

Beispiel 6

Das Verfahren von Beispiel 1 wurde wiederholt, man verwendete jedoch statt Toluol 100 Gew.-Teile t.-Butylbenzol. Der Restgehalt an tert.-Butylbenzol betrug 10,3 Gew.-%, das Verhältnis von ortho- zu para-Chlor-t.-butylbenzol betrug o/p = 0,22.

Beispiel 7

Das Verfahren von Beispiel 1 wurde wiederholt. Man gab jedoch 0,005 Gew.-Teile des Co-Katalysators der Formel

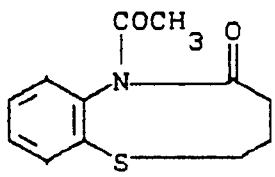

(6-Acetyl-3,4-dihydro-2H-1,6-
benzothiazocin-5(6H)-on

zu. Der Resttoluolgehalt betrug 4,2 Gew.-%, das o/p-Verhältnis betrug o/p = 0,90.

Beispiel 8

Das Verfahren von Beispiel 1 wurde wiederholt. Man gab jedoch 0,0045 Gew.-Teile des Co-Katalysators der Formel

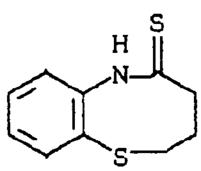

(3,4-Dihydro-2H-1,6-benzo-
thiazocin-5(6H)-thion)

zu. Der Resttoluolgehalt betrug 3,5 Gew.-%, das o/p-Verhältnis 1,00.

Beispiel 9

Das Verfahren von Beispiel 1 wurde wiederholt. Man gab jedoch 0,005 Gew.-Teile des Co-Katalysators der Formel

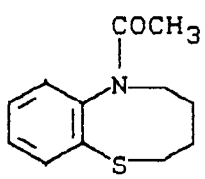

(6-Acetyl-3,4,5,6-tetrahydro-
thiazocin)

zu. Der Resttoluolgehalt betrug 4,1 Gew.-%, das o/p-Verhältnis 0,93.

Beispiel 10

Das Verfahren von Beispiel 1 wurde wiederholt. Man gab jedoch 0,0061 Gew.-Teile des Co-Katalysators der Formel

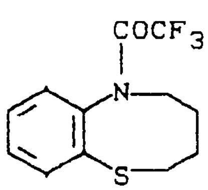

(6-Trifluoracetyl-3,4,5,6-
tetrahydro-2H-1,6-benzo-
thiazocin)

zu. Der Resttoluolgehalt betrug 3,3 Gew.-%, das o/p-Verhältnis war 0,98.

Beispiel 11

Das Verfahren von Beispiel 1 wurde wiederholt. Man gab jedoch 0,0058 Gew.-Teile des Co-Katalysators der Formel

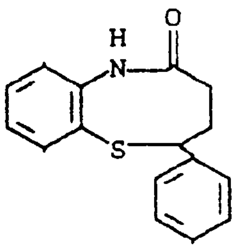

(2-Phenyl-3,4-dihydro-2H-1,6-benzothiazocin-5(6H)-on)

zu. Der Resttoluolgehalt betrug 5,4 Gew.-%, das o/p-Verhältnis 1,02.

Beispiel 12

Das Verfahren von Beispiel 1 wurde wiederholt. Man gab jedoch 0,0045 Gew.-Teile des Co-Katalysators der Formel

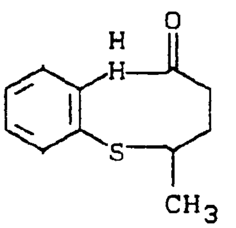

(2-Methyl-3,4-dihydro-2H-1,6-benzothiazocin-5(6H)-on)

zu. Der Resttoluolgehalt betrug 8,9 Gew.-%, das o/p-Verhältnis 1,10.

Beispiel 13

Das Verfahren von Beispiel 1 wurde wiederholt. Man gab jedoch 0,0112 Gew.-Teile des Co-Katalysators der Formel

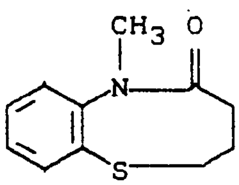

(6-Methyl-3,4-dihydro-2H-1,6-benzothiazocin-5(6H)-on)

zu. Der Resttoluolgehalt betrug 3,5 Gew.-%, das o/p-Verhältnis 1,03.

Beispiel 14

Das Verfahren von Beispiel 1 wurde wiederholt. Man verwendete jedoch nur 0,0011 Gew.-Teile des dortigen Co-Katalysators. Der Resttoluolgehalt betrug 4,0 Gew.-%, das o/p-Verhältnis 0,78.

Beispiel 15

Das Verfahren von Beispiel 1 wurde wiederholt. Man verwendete jedoch nur 0,00055 Gew.-Teile des dortigen Co-Katalysators. Der Resttoluolgehalt betrug 4,4 Gew.-%, das o/p-Verhältnis 0,79.

Beispiel 16

Das Verfahren von Beispiel 2 wurde wiederholt. Man verwendete jedoch nur 0,0011 Gew.-Teile des dortigen Co-Katalysators. Der Resttoluolgehalt betrug 8,4 Gew.-%, das o/p-Verhältnis 1,05.

Beispiel 17

Das Verfahren von Beispiel 13 wurde wiederholt. Man verwendete jedoch nur 0,0045 Gew.-Teile des dortigen Co-Katalysators. Der Resttoluolgehalt betrug 7,3 Gew.-%, das o/p-Verhältnis 1,06.

Beispiel 18

Das Verfahren von Beispiel 13 wurde wiederholt. Man verwendete jedoch nur 0,0012 Gew.-Teile des dortigen Co-Katalysators. Der Resttoluolgehalt betrug 5,0 Gew.-%, das o/p-Verhältnis 1,00.

Beispiel 19

Das Verfahren von Beispiel 9 wurde wiederholt. Man verwendete jedoch nur 0,0012 Gew.-Teile des dortigen Co-Katalysators. Der Resttoluolgehalt betrug 5,4 Gew.-%, das o/p-Verhältnis 1,10.

Beispiel 20

Das Verfahren von Beispiel 7 wurde wiederholt. Man verwendete jedoch nur 0,0013 Gew.-Teile des dortigen Co-Katalysators. Der Resttoluolgehalt betrug 5,2 Gew.-%, das o/p-Verhältnis betrug 0,88.

Beispiel 21 (Vergleichsbeispiel)

In 100 Gew.-Teilen Toluol werden 0,07 Gew.-Teile $FeCl_3$ und 0,29 Gew.-Teile des nach der EP 0 173 222-Vorschrift hergestellten Phenoxathiinderivats gelöst. Man leitete bei 50°C ca. 94 Mol-% Chlor, bezogen auf Toluol, gasförmig unter Rühren ein. Der Restgehalt an Toluol betrug 7,9 %, das o/p-Verhältnis 0,88.

Beispiel 22 (Vergleichsbeispiel)

Das Verfahren des Beispiels 21 wurde wiederholt. In 100 Gew.-Teilen Toluol wurden 0,0175 Gew.-Teile $FeCl_3$ und 0,008 Gew.-Teile des nach der EP 0 173 222-Vorschrift hergestellten Phenoxathiinderivats gelöst. Man leitete bei 50°C ca. 94 Mol-% $Cl_2$, bezogen auf Toluol, gasförmig unter Rühren ein. Der Restgehalt an Toluol betrug 6,4 Gew.-%, das o/p-Verhältnis 1,26.

Beispiel 23 (Vergleichsbeispiel)

Das Verfahren des Beispiels 21 wurde wiederholt. In 100 Gew.-Teilen Toluol wurden 0,0175 Gew.-Teile $FeCl_3$ und 0,0065 Gew.-Teile des in Beispiel 4 der US-P 4 031 147 genannten Co-Katalysators der Formel

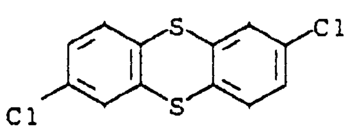

(2,7-Dichlorthianthren)

gelöst. Man erhitzte auf 50°C und leitete unter Rühren ca. 94 Mol-% $Cl_2$, bezogen auf Toluol, gasförmig ein. Der Resttoluolgehalt betrug 6,7 Gew.-%, das o/p-Verhältnis 1,55.

**Patentansprüche**

1. Verfahren zur Kernchlorierung von aromatischen Kohlenwasserstoffen der Formel

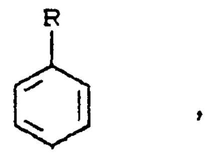

worin
R geradkettiges oder verzweigtes $C_1$-$C_{12}$-Alkyl oder $C_3$-$C_8$-Cycloalkyl bedeutet,
in Gegenwart von Friedel-Crafts-Katalysatoren und in Gegenwart von Co-Katalysatoren in flüssiger Phase, das dadurch gekennzeichnet ist, daß man als Co-Katalysatoren 1,6-Benzo-thiazocine einsetzt.

2. Verfahren nach Anspruch 1, dadurch gekennzeichnet, daß man 1,6-Benzo-thiazocine der folgenden Formel einsetzt

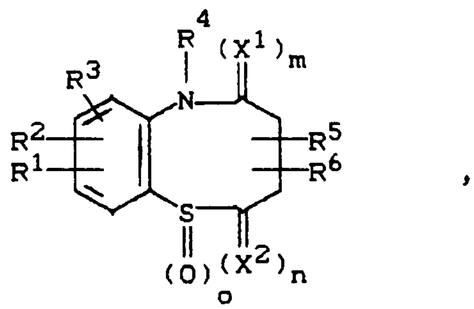

in der
$R^1$ und $R^2$ unabhängig voneinander Wasserstoff, Hydroxy, Amino, Cyano, Halogen, Nitro, Alkylsulfonyl, Phenylsulfonyl, Alkylsulfoxyl, Phenylsulfoxyl, Tosyl, Mercapto, Carboxyl, Halogenocarbonyl, Carboxyamid, Alkoxycarbonyl, Thiocarboxyamid, Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino bedeuten,
$R^3$ für Wasserstoff oder Chlor steht und weiterhin mit einem der Reste $R^1$ oder $R^2$ und gemeinsam mit den substituierten C-Atomen einen annellierten gesättigten, ungesättigten oder aromatischen isocyclischen oder heterocyclischen 5-8-Ring bilden kann,
$R^4$ Wasserstoff, Alkyl, Aryl, Heteroaryl, Acyl, Thioacyl, Halogencarbonyl oder Alkoxycarbonyl bedeutet,
$X^1$ und $X^2$ unabhängig voneinander für doppelt gebundenen Sauerstoff, Schwefel oder $R^7$-substituierten Stickstoff stehen wobei $R^7$ den Bedeutungsumfang von $R^4$ mit Ausnahme von Wasserstoff hat,
m, n und o unabhängig voneinander den Wert 0 oder 1 annehmen können und
$R^5$ und $R^6$ unabhängig voneinander an einem oder an zwei der zwischen dem S- und dem N-Atom im 8-Ring befindlichen C-Atome stehen können, sofern diese C-Atome nicht durch $X^1$ bzw. $X^2$ besetzt sind, und den Bedeutungsumfang von $R^1$ bzw. $R^2$ haben, wobei bei benachbarter Substitution auch mit den substituierten C-Atomen ein gesättigter, ungesättigter oder aromatischer isocyclischer oder heterocyclischer 5-8-Ring gebildet werden kann und wobei weiterhin der Bedeutungsumfang des doppelt gebundenen Sauerstoffs oder Schwefels angenommen werden kann.

3. Verfahren nach Anspruch 2, dadurch gekennzeichnet, daß man 1,6-Benzo-thiazocine der Formeln

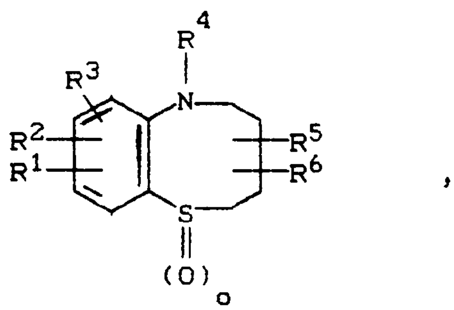

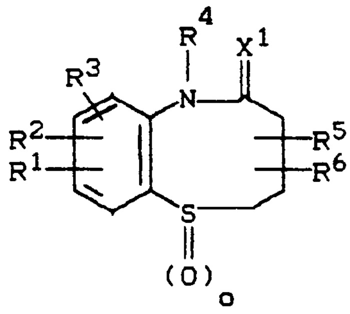

oder

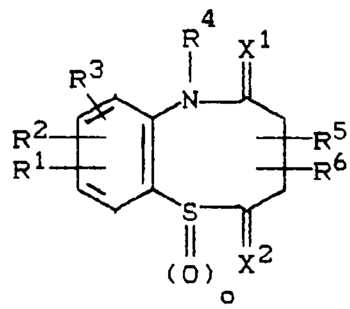

einsetzt, in denen
$R^1$, $R^2$, $R^3$, $R^4$, $R^5$, $R^6$, $X^1$, $X^2$ und o den in Anspruch 2 genannten Bedeutungsumfang haben.

4. Verfahren nach Anspruch 3, dadurch gekennzeichnet, daß man 1,6-Benzo-thiazocine der Formeln

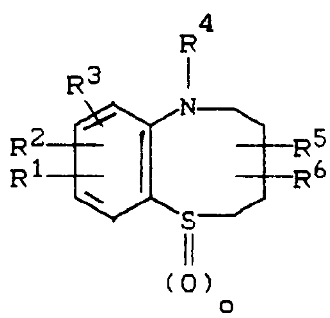

oder

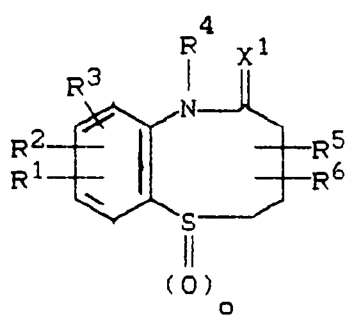

einsetzt, in denen
$R^1$, $R^2$, $R^3$, $R^4$, $R^5$, $R^6$, $X^1$ und o den in Anspruch 2 genannten Bedeutungsumfang haben.

5. Verfahren nach Anspruch 4, dadurch gekennzeichnet, daß man 1,6-Benzo-thiazocine der Formel

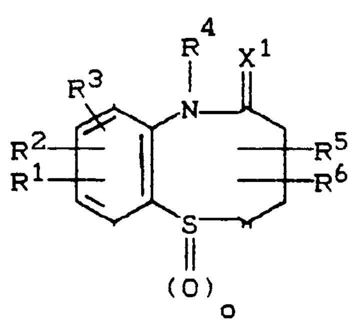

einsetzt, in denen
$R^1$, $R^2$, $R^3$, $R^4$, $R^5$, $R^6$, $X^1$, und o den in Anspruch 2 genannten Bedeutungsumfang haben.

6. Verfahren nach Anspruch 2, dadurch gekennzeichnet, daß o den Wert Null annimmt.

7. Verfahren nach Anspruch 2, dadurch gekennzeichnet, daß an die Stelle von $R^1$, $R^2$ und $R^3$ die Reste $R^{11}$, $R^{12}$ bzw. $R^{13}$ treten, von denen
$R^{11}$ und $R^{12}$ unabhängig voneinander Wasserstoff, Halogen, Nitro, Alkyl, Aryl, Alkoxy, Aryloxy, Acyloxy, Alkylthio, Arylthio, Acyl oder Thioacyl bedeuten und
$R^{13}$ für Wasserstoff oder Chlor steht und weiterhin mit einem der Reste $R^{11}$ oder $R^{12}$ und gemeinsam mit den substituierten C-Atomen einen annellierten, gesättigten, isocyclischen 5-7-Ring oder einen annellierten Benzolring bilden kann,
und bevorzugt dadurch gekennzeichnet, daß an die Stelle von $R^{11}$, $R^{12}$ und $R^{13}$ die Reste $R^{21}$, $R^{22}$ bzw. $R^{23}$ treten, von denen
$R^{21}$ und $R^{22}$ unabhängig voneinander Wasserstoff, $C_1$-$C_4$-Alkyl, $C_1$-$C_4$-Alkoxy, Fluor oder Chlor bedeuten und $R^{23}$ für Wasserstoff oder Chlor steht und weiterhin mit einem der Reste $R^{21}$ und $R^{22}$ und gemeinsam mit den substituierten C-Atomen einen annellierten Cyclopentan-, Cyclohexan- oder Benzolring bilden kann.

8. Verfahren nach Anspruch 2, dadurch gekennzeichnet, daß an die Stelle von $R^4$ der Rest $R^{14}$ tritt, der die Bedeutung Wasserstoff, $C_1$-$C_4$-Alkyl, Phenyl, $C_1$-$C_4$-Acyl oder $C_2$-$C_4$-Alkoxycarbonyl hat,
und besonders dadurch gekennzeichnet, daß an die Stelle von $R^{14}$ der Rest $R^{24}$ mit der Bedeutung Wasserstoff, $C_1$-$C_2$-Alkyl, Benzyl, Phenyl, Acetyl, Trifluoracetyl, Chloracetyl, Chlorcarbonyl oder Propionyl tritt.

9. Verfahren nach Anspruch 2, dadurch gekennzeichnet, daß an die Stelle von $R^5$ und $R^6$ die Reste $R^{15}$ bzw. $R^{16}$ treten, die die Bedeutung Wasserstoff, $C_1$-$C_4$-Alkyl, $C_1$-$C_4$-Alkoxy, $C_1$-$C_4$-Acyl, Phenyl, Fluor oder Chlor haben und wobei weiterhin bei benachbarter Substitution beide Reste gemeinsam mit den substituierten C-Atomen einen Cyclopentan-, Cyclohexan- oder Benzolring bilden können.

10. Verfahren nach Ansprüchen 1 und 2, dadurch gekennzeichnet, daß die Menge an eingesetztem Co-Katalysator 0,0001 bis 0,5 Gew.-%, bevorzugt 0,0005 bis 0,1 Gew.-%, besonders bevorzugt 0,0005 bis 0,0075 Gew.-%, bezogen auf den eingesetzten aromatischen Kohlenwasserstoff, beträgt.

## Claims

1. Process for the ring chlorination of aromatic hydrocarbons of the formula

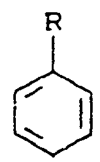

,

in which
R denotes straight-chain or branched $C_1$-$C_{12}$-alkyl or $C_3$-$C_8$-cycloalkyl
in the presence of Friedel-Crafts catalysts and in the presence of co-catalysts in liquid phase, which is characterized in that the co-catalysts used are 1,6-benzothiazocins.

2. Process according to Claim 1, characterized in that 1,6-benzothiazocins of the following formula are used

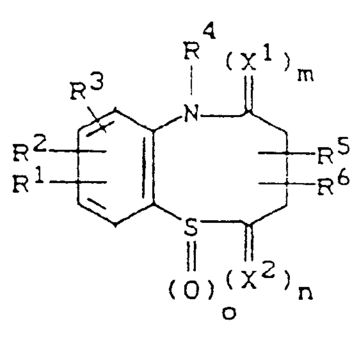

,

in which
$R^1$ and $R^2$, independently of one another, denote hydrogen, hydroxyl, amino, cyano, halogen, nitro, alkylsulphonyl, phenylsulphonyl, alkylsulphoxyl, phenylsulphoxyl, tosyl, mercapto, carboxyl, halogenocarbonyl, carboxyamide, alkoxycarbonyl, thiocarboxyamide, alkyl, aryl, heteroaryl, alkoxy, aryloxy, heteroaryloxy, acyloxy, alkylthio, arylthio, heteroarylthio, acylthio, acyl, thioacyl or acylamino,
$R^3$ represents hydrogen or chlorine and furthermore can form a fused saturated, unsaturated or aromatic isocyclic or heterocyclic 5- to 8-membered ring with one of the radicals $R^1$ or $R^2$ and together with the substituted carbon atoms,
$R^4$ denotes hydrogen, alkyl, aryl, heteroaryl, acyl, thioacyl, halogenocarbonyl or alkoxycarbonyl,
$X^1$ and $X^2$, independently of one another, representdoubly bound oxygen, sulphur or $R^7$-substituted nitrogen, $R^7$ having the range of meaning of $R^4$ with the exception of hydrogen,
m, n and o, independently of one another, can adopt the value 0 or 1, and
$R^5$ and $R^6$, independently of one another, can be located on one or two of the carbon atoms between the S and N atom in the 8-membered ring, unless these carbon atoms are occupied by $X^1$ or $X^2$, and have the range of meaning of $R^1$ and $R^2$, where in the case of vicinal substitution they can also form a saturated, unsaturated or aromatic isocyclic or heterocyclic 5- to 8-membered ring with the substituted carbon atoms and where furthermore they can adopt the range of meaning of the doubly bound oxygen or sulphur.

3. Process according to Claim 2, characterized in that 1,6-benzothiazocins of the formula

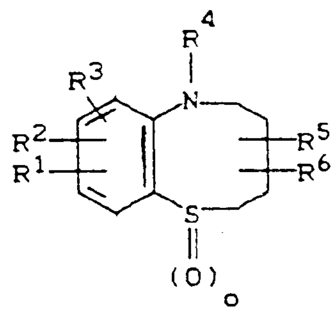 ,

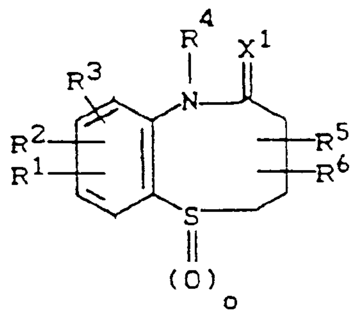

or

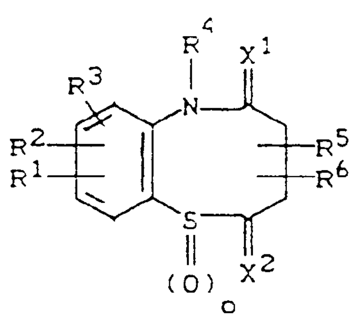

are used in which
$R^1$, $R^2$, $R^3$, $R^4$, $R^5$, $R^6$, $X^1$, $X^2$ and o have the range of meaning given in Claim 2.

4. Process according to Claim 3, characterized in that 1,6-benzothiazocins of the formula

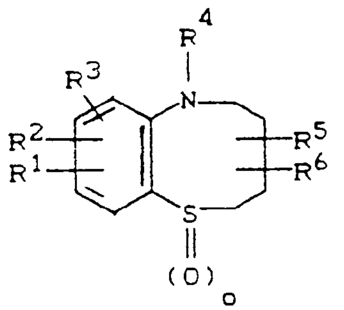

or

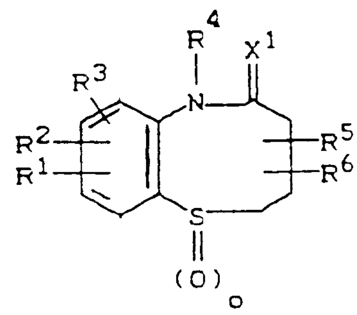

are used in which

$R^1$, $R^2$, $R^3$, $R^4$, $R^5$, $R^6$, $X^1$ and o have the range of meaning given in Claim 2.

5. Process according to Claim 4, characterized in that 1,6-benzothiazocins of the formula

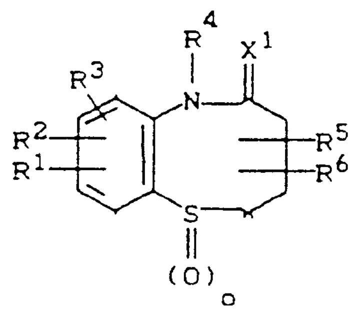

are used in which

$R^1$, $R^2$, $R^3$, $R^4$, $R^5$, $R^6$, $X^1$ and o have the range of meaning given in Claim 2.

6. Process according to Claim 2, characterized in that o adopts the value zero.

7. Process according to Claim 2, characterized in that the place of $R^1$, $R^2$ and $R^3$ is taken by the radicals $R^{11}$, $R^{12}$ and $R^{13}$, of which

$R^{11}$ and $R^{12}$, independently of one another, denote hydrogen, halogen, nitro, alkyl, aryl, alkoxy, aryloxy, acyloxy, alkylthio, arylthio, acyl or thioacyl and

$R^{13}$ represents hydrogen or chlorine and furthermore can form a fused, saturated, isocyclic 5- to 7-membered ring or a fused benzene ring with one of the radicals $R^{11}$ or $R^{12}$ and together with the substituted carbon atoms, and preferably characterized in that the place of $R^{11}$, $R^{12}$ and $R^{13}$ is taken by the radicals $R^{21}$, $R^{22}$ and $R^{23}$, of which

$R^{21}$ and $R^{22}$, independently of one another, denote hydrogen, $C_1$-$C_4$-alkyl, $C_1$-$C_4$-alkoxy, fluorine or chlorine and $R^{23}$ represents hydrogen or chlorine and furthermore can form a fused cyclopentane, cyclohexane or Benzene ring with one of the radicals $R^{21}$ and $R^{22}$ and together with the substituted carbon atoms.

8. Process according to Claim 2, characterized in that the place of $R^4$ is taken by $R^{14}$ denoting hydrogen, $C_1$-$C_4$-alkyl, phenyl, $C_1$-$C_4$-acyl or $C_2$-$C_4$-alkoxycarbonyl

and particularly characterized in that the place of $R^{14}$ is taken by the radical $R^{24}$ denoting hydrogen, $C_1$-$C_2$-alkyl, benzyl, phenyl, acetyl, trifluoroacetyl, chloroacetyl, chlorocarbonyl or propionyl.

9. Process according to Claim 2, characterized in that the place of $R^5$ and $R^6$ is taken by the radicals $R^{15}$ and $R^{16}$ denoting hydrogen, $C_1$-$C_4$-alkyl, $C_1$-$C_4$-alkoxy, $C_1$-$C_4$-acyl, phenyl, fluorine or chlorine and where in the case of vicinal substitution both radicals can furthermore form a cyclopentane, cyclohexane or benzene ring together with the substituted carbon atoms.

10. Process according to Claims 1 and 2, characterized in that the amount of co-catalyst used is 0.0001 to 0.5% by weight, preferably 0.0005 to 0.1% by weight, particularly preferably 0.0005 to 0.0075% by weight, relative to the aromatic hydrocarbon used.

## Revendications

1. Procédé de chloration dans le noyau d'hydrocarbures aromatiques de formule

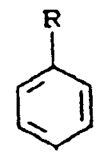

dans laquelle

R est un groupe alkyle en $C_1$ à $C_{12}$ à chaîne droite ou ramifiée ou un groupe cycloalkyle en $C_3$ à $C_8$,

en présence de catalyseurs de Friedel-Crafts et en présence de co-catalyseurs en phase liquide, qui est caractérisé en ce qu'on utilise comme co-catalyseurs des 1,6-benzothiazocines.

2. Procédé suivant la revendication 1, caractérisé en ce qu'on utilise des 1,6-benzothiazocines de formule suivante

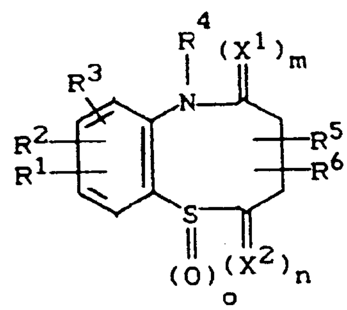

dans laquelle

$R^1$ et $R^2$ représentent, indépendamment l'un de l'autre, l'hydrogène, un groupe hydroxy, amino, cyano, un halogène, un groupe nitro, alkylsulfonyle, phénylsulfonyle, alkylsulfoxyle, phénylsulfoxyle, tosyle, mercapto, carboxyle, halogénocarbonyle, carboxyamido, alkoxycarbonyle, thiocarboxyamido, alkyle, aryle, hétéro-aryle, alkoxy, aryloxy, hétéro-aryloxy, acyloxy, alkylthio, arylthio, hétéro-arylthio, acylthio, acyle, thioacyle ou acylamino,

$R^3$ est l'hydrogène ou le chlore et peut en outre former avec l'un des restes $R^1$ et $R^2$ et conjointement avec les atomes de carbone substitués un noyau pentagonal à octogonal isocyclique ou hétérocyclique condensé saturé, non saturé ou aromatique,

$R^4$ est l'hydrogène, un groupe alkyle, aryle, hétéro-aryle, acyle, thioacyle, halogénocarbonyle ou alkoxycarbonyle,

$X^1$ et $X^2$ représentent, indépendamment l'un de l'autre, de l'azote à substituant $R^7$, du soufre ou de l'oxygène attaché par une double liaison, $R^7$ entrant dans le cadre de la définition de $R^4$ excepté l'hydrogène,

m, n et o peuvent avoir, indépendamment les uns des autres, la valeur 0 ou 1 et

$R^5$ et $R^6$ peuvent se trouver, indépendamment l'un de l'autre, sur un ou deux des atomes de carbone se trouvant entre l'atome de soufre et l'atome d'azote dans le noyau octogonal, dans la mesure où ces atomes de carbone ne sont pas occupés par $X^1$ ou $X^2$, et rentrent dans le cadre de la définition de $R^1$ et, respectivement, $R^2$, et dans le cas d'une substitution adjacente, un noyau saturé, non saturé ou aromatique isocyclique ou hétérocyclique pentagonal à octogonal peut aussi être formé avec les atomes de carbone substitués et en outre, le cadre de la définition de l'oxygène ou du soufre attaché par une double liaison peut être adopté.

3. Procédé suivant la revendication 2, caractérisé en ce qu'on utilise des 1,6-benzothiazocines de formules

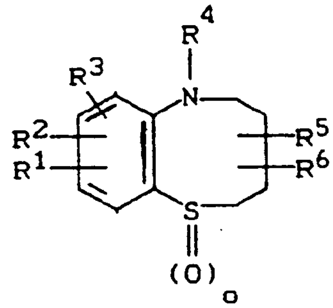 ,

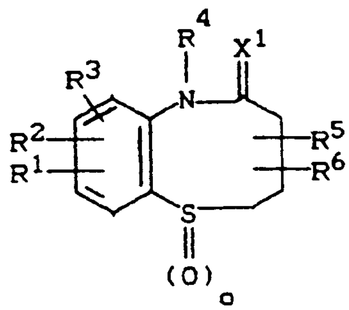

ou

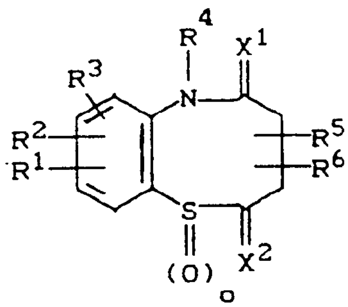

dans lesquelles
$R^1$, $R^2$, $R^3$, $R^4$, $R^5$, $R^6$, $X^1$, $X^2$ et o ont la définition mentionnée dans la revendication 2.

4. Procédé suivant la revendication 3, caractérisé en ce qu'on utilise des 1,6-benzothiazocines de formules

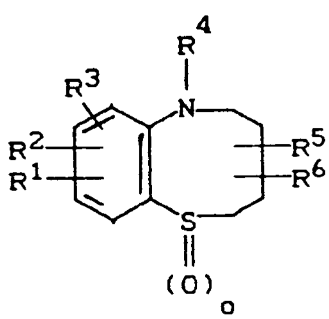

ou

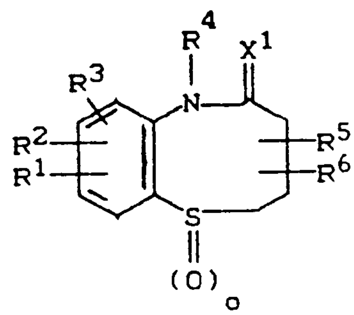

dans lesquelles
$R^1$, $R^2$, $R^3$, $R^4$, $R^5$, $R^6$, $X^1$ et o ont la définition donnée dans la revendication 2.

5. Procédé suivant la revendication 4, caractérisé en ce qu'on utilise des 1,6-benzothiazocines de formule

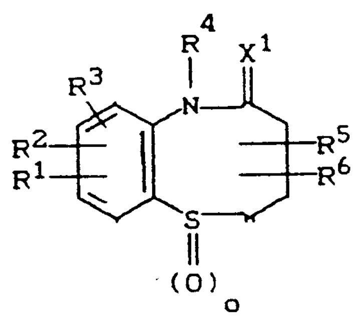

dans laquelle
$R^1$, $R^2$, $R^3$, $R^4$, $R^5$, $R^6$, $X^1$ et o ont la définition indiquée dans la revendication 2.

6. Procédé suivant la revendication 2, caractérisé en ce que o prend la valeur zéro.

7. Procédé suivant la revendication 2, caractérisé en ce que $R^1$, $R^2$ et $R^3$ sont remplacés par les restes $R^{11}$, $R^{12}$ et, respectivement, $R^{13}$ parmi lesquels
$R^{11}$ et $R^{12}$ représentent, indépendamment l'un de l'autre, l'hydrogène, un halogène, un groupe nitro, alkyle, aryle, alkoxy, aryloxy, acyloxy, alkylthio, arylthio, acyle ou thioacyle et
$R^{13}$ représente l'hydrogène ou le chlore et peut en outre former avec l'un des restes $R^{11}$ et $R^{12}$ ou conjointement avec les atomes de carbone substitués un noyau pentagonal à heptagonal condensé saturé isocyclique ou un noyau benzénique condensé,
caractérisé de préférence en ce que $R^{11}$, $R^{12}$ et $R^{13}$ sont remplacés, respectivement, par les restes $R^{21}$, $R^{22}$ et $R^{23}$ parmi lesquels
$R^{21}$ et $R^{22}$ représentent, indépendamment l'un de l'autre, l'hydrogène, un groupe alkyle en $C_1$ à $C_4$, alkoxy en $C_1$ à $C_4$, du fluor ou du chlore et
$R^{23}$ représente l'hydrogène ou le chlore et peut en outre former avec l'un des restes $R^{21}$ et $R^{22}$ et conjointement avec les atomes substitués de carbone un noyau de cyclopentane, de cyclohexane ou de benzène condensé.

8. Procédé suivant la revendication 2, caractérisé en ce que $R^4$ est remplacé par le reste $R^{14}$ qui a pour définition l'hydrogène, un groupe alkyle en $C_1$ à $C_4$, phényle, acyle en $C_1$ à $C_4$ ou alkoxycarbonyle en $C_2$ à $C_4$, et caractérisé en particulier en ce que le reste $R^{14}$ est remplacé par le reste $R^{24}$ ayant pour définition l'hydrogène, un groupe alkyle en $C_1$ ou $C_2$, benzyle, phényle, acétyle, trifluoracétyle, chloracétyle, chlorocarbonyle ou propionyle.

9. Procédé suivant la revendication 2, caractérisé en ce que les restes $R^5$ et $R^6$ sont remplacés par les restes $R^{15}$ et, respectivement, $R^{16}$ qui ont pour définition l'hydrogène, un groupe alkyle en $C_1$ à $C_4$, alkoxy en $C_1$ à $C_4$, acyle en $C_1$ à $C_4$, phényle, le fluor ou le chlore et en outre, en cas de substitution adjacente, les deux restes peuvent former conjointement avec les atomes substitués de carbone un noyau de cyclopentane, de cyclohexane ou de benzène.

10. Procédé suivant les revendications 1 et 2, caractérisé en ce que la quantité utilisée de co-catalyseur va de 0,0001 à 0,5 % en poids, de préférence de 0,0005 à 0,1 % en poids, notamment de 0,0005 à 0,0075 % en poids, par rapport à l'hydrocarbure aromatique utilisé.